# EUROPEAN PATENT SPECIFICATION

(11) **EP 3 829 501 B1**
(45) Date of publication and mention of the grant of the patent: **31.12.2025**
(21) Application number: 19843323.7
(22) Date of filing: 05.08.2019
(51) Int. Cl.: A61F 9/007

(54) **RETINAL IMPLANTATION DEVICE**
RETINAIMPLANTAT
DISPOSITIF D'IMPLANTATION RÉTINIEN

(30) Priority: 03.08.2018 US 201862714280 P
(43) Date of publication of application: 09.06.2021
(73) Proprietor: The Johns Hopkins University, Baltimore, Maryland 21218 (US)
(72) Inventor: SINGH, Mandeep, Baltimore, Maryland 21209 (US); WOLFE, Kevin, Lutherville, Maryland 21093 (US); KANG, Jin Ung, Glenelg, Maryland 21737 (US); PERRINO, Kathleen, Laurel, Maryland 20723 (US); TIMM, Andrea Cascio, Laurel, Maryland 20723 (US)
(74) Representative: Murgitroyd & Company
(86) International application number: PCT/US2019/045074
(87) International publication number: WO 2020/028892

(56) References cited:
- EP-A2- 0 138 564
- WO-A1-2012/142318
- WO-A1-94/21205
- WO-A1-96/26759
- US-A- 5 962 027
- US-A1- 2010 255 061
- US-A1- 2013 253 438
- US-A1- 2014 303 544
- US-A1- 2014 303 544
- US-B2- 8 057 483
- US-B2- 8 057 483

## Description

### FIELD OF THE INVENTION

The present invention relates to a retinal implantation device.

### BACKGROUND OF THE INVENTION

Photoreceptor transplantation is a potential treatment for adults and children who have lost vision from retinal degenerative diseases, including age-related macular degeneration (AMD) and inherited retinal degenerations. This treatment strategy has been shown to be effective in animal models. We are developing protocols and enabling devices for photoreceptor generation from stem cells.

The major challenge for effective human therapy to be developed is in delivery. Current delivery devices all rely on accessing the target zone, which is the subretinal space, via the trans-retinal approach (i.e. ab interno approach). This entails making a surgical incision in the retina, in order to gain access to the target space. This retinal incision is associated with a high rate of complications including bleeding, scarring, retinal detachment, and off target delivery of the cells due to reflux into other ocular compartments.

Accordingly, there is a need in the art for a device for implantation of cells at the retina. Prior art examples are described in US 2014/303544 A1 (HAFFNER) and US Patent 5,962,027 (HUGHES).

US 2014/303544 A1 dislcoses a drug delivery ocular implant comprising an outer shell shaped to define an interior lumen and the lumen comprising an active drug.

US 5,962,027 discloses an instrument for implanting a structure into the eye and comprising an elongate tube having a flat cross-section with a top and a bottom and opposing sides.

### SUMMARY OF THE INVENTION

The present invention seeks to protect a device for the delivery of material in a retinal space of an eve as defined in claim 1, and the optional dependent claims.

The foregoing needs are met, to a great extent, by the present invention which provides a device for delivery of material including a pair of thin, elongate strips of flexible material. The device includes a thin layer of elastic material surrounding the pair of thin, elongate strips of flexible material. The pair of thin, elongate strips of flexible material and thin layer of elastic material surrounding the pair of thin, elongate strips of flexible material form a flexible, expandable tube. An elongate lumen extends between the pair of thin, elongate strips of flexible material.

In accordance with the present invention, the pair of thin, elongate strips of flexible material are formed from a flexible material that allows the device to curve along the subretinal space. More particularly, the pair of thin, elongate strips are formed from polyimide. The thin layer of elastic material is formed from latex. The device includes fixation holes along each edge of the strips of flexible material (figure attached) that are used to secure the device to the eye during the insertion procedure. The elongate lumen is configured for dispensing material or cells through the device. A geometry of the device is configured such that the device is extremely flexible in one bending direction, but more rigid in the other direction. The device can include an optical coherence tomography sensor for intraretinal visualization to guide and create a subretinal space where the cells will be deposited. The OCT-sensor allows a user to determine depth of penetration of the device at entry and detect retinal layers. A distal end of the pair of thin, elongate strips of flexible material has a rounded shape. The device is configured to move through a subretinal space of an eye.

In accordance with the present invention, the pair of thin, elongate strips of flexible material are formed from a material that allows the passage of objects between the pair of thin, elongate strips of flexible material. The device includes a lubricating material disposed between the pair of thin, elongate strips of flexible material to facilitate smooth passage and prevent adhesion of the material to be delivered. The device can also include a hub for facilitating the injection of material or cells to the retina. The hub can include a reservoir for the material or cells. The reservoir can take the form of a syringe. A microfluidic platform is included to lift the product of the injector using a "no-touch" technique.

In accordance with an aspect of the present invention not defined in the claims, a method of delivering material to the retina includes accessing the subretinal space. The method includes visualizing the subretinal space. The method also includes depositing material in the subretinal space using a pair of thin, elongate strips of flexible material. There is a thin layer of elastic material surrounding the pair of thin, elongate strips of flexible material to form a flexible, expandable tube, wherein an elongate lumen extends between the pair of thin, elongate strips of flexible material for depositing the material.

In accordance with the present invention not defined in the claims, the method includes visualizing the subretinal space using an optical coherence tomography sensor that is mounted or placed at or near the tip of the device. The OCT-sensor can also be integrated into a guide needle. The OCT-integrated guide needle opens a passage and creates a subretinal bleb. The method also includes accessing the subretinal space via an incision in the sclera and choroid.

### BRIEF DESCRIPTION OF THE DRAWING

The accompanying drawings provide visual representations, which will be used to more fully describe the representative embodiments disclosed herein and can be used by those skilled in the art to better understand them and their inherent advantages. In these drawings, like reference numerals identify corresponding elements and:
FIG. 1A illustrates a partially sectional view of an OCT-sensor integrated guide needle, according to an embodiment of the present invention.
FIG. 1B illustrates a partially sectional view of an entry point and a target point, according to an embodiment of the present invention.
FIG. 1C illustrates a side view of an injection cartridge tip, according to an embodiment of the present invention.
FIG. 2A illustrates a perspective view of a device for delivery of cells to the retina, according to an embodiment of the present invention.
FIG. 2B illustrates a top-down view of a device for delivery of cells to the retina, according to an embodiment of the present invention.
FIG. 2C illustrates an enlarged view of a distal end of the device for delivery of cells to the retina of FIG. 2B, according to an embodiment of the present invention.
FIG. 2D illustrates a front view of a distal end of the device for delivery of cells to the retina of FIG. 2B, according to an embodiment of the present invention.
FIG. 2E illustrates a side view of a device for delivery of cells to the retina, according to an embodiment of the present invention.
FIG. 2F illustrates an enlarged view of a distal end of the device for delivery of cells to the retina of FIG. 2E, according to an embodiment of the present invention.
FIG. 3A illustrates a perspective view of a device for delivery of cells to the retina, according to an embodiment of the present invention.
FIG. 3B illustrates an enlarged view of a distal end of the device for delivery of cells to the retina of FIG. 3A, according to an embodiment of the present invention.
FIG. 3C illustrates an enlarged view of a distal end of the device for delivery of cells to the retina of FIG. 3B, according to an embodiment of the present invention.
FIGS. 4A and 4B illustrate views a device for delivery of material or cells to the retina, according to an embodiment of the present invention.
FIG. 5A illustrates a top down view of a bottom layer of a device for delivery of material or cells to the retina, according to an embodiment of the present invention. FIG. 5B illustrates a top down view of a top layer of a device for delivery of material or cells to the retina, according to an embodiment of the present invention.
FIG. 6A illustrates a top down view of a device for delivery of material or cells to the retina, according to an embodiment of the present invention. FIG. 6B illustrates an end view of the device for delivery illustrated in FIG. 6A.
FIGS. 7A and 7B illustrate perspective views of a device for delivery of material or cells to the retina, according to an embodiment of the present invention.
FIG. 8 illustrates a perspective view of a device for delivery of material or cells to the retina, according to an embodiment of the present invention.

### DETAILED DESCRIPTION

The presently disclosed subject matter now will be described more fully hereinafter with reference to the accompanying Drawings, in which some, but not all embodiments of the inventions are shown. Like numbers refer to like elements throughout.

The present invention is directed to a device for the access and delivery of cells or other materials to the subretinal space. The device of the present invention accesses the subretinal space via the trans-scleral side. This is an ab externo approach. The cell delivery device includes two stacked layers, surrounded by a flexible outer surface. The device is configured to be flexible, such that it conforms to the natural curvature of the eye as it is advanced to the subretinal space. After the device is in place, the flexible outer surface is configured to protect the delicate tissue of the retina and retinal pigment epithelium and choroid, while there is also easy passage of the material or cells to be delivered between the two stacked layers. The device of the present invention is configured to avoid or ameliorate all these complications using an innovative device for the delivery of retinal stem cells, or other materials, to the subretinal space.

The device of the present invention includes a number of novel features. One such feature addresses the current inability to visualize the depth of penetration at the entry phase, because the sclera is completely opaque. In addition, this trans-sclera approach prevents the use of standard surgical microscope mounted intraoperative OCT systems for the visualization of retinal tissue through pupil. To circumvent this limitation, the present invention includes an OCT sensor directly integrated into a guide needle to allow for visualization of the subretinal space during the subretinal space opening process. Another feature addresses that the propagation trajectory is a curved line and no device exists to safely navigate this propagation tunnel. The present invention includes a novel flexible cannula and injector system to safely navigate the propagation tunnel. Plunger systems which exert force against the biologic product risk damaging it. Therefore, the present invention includes a microfluidic platform to lift the product of the injector using a "no-touch" technique.

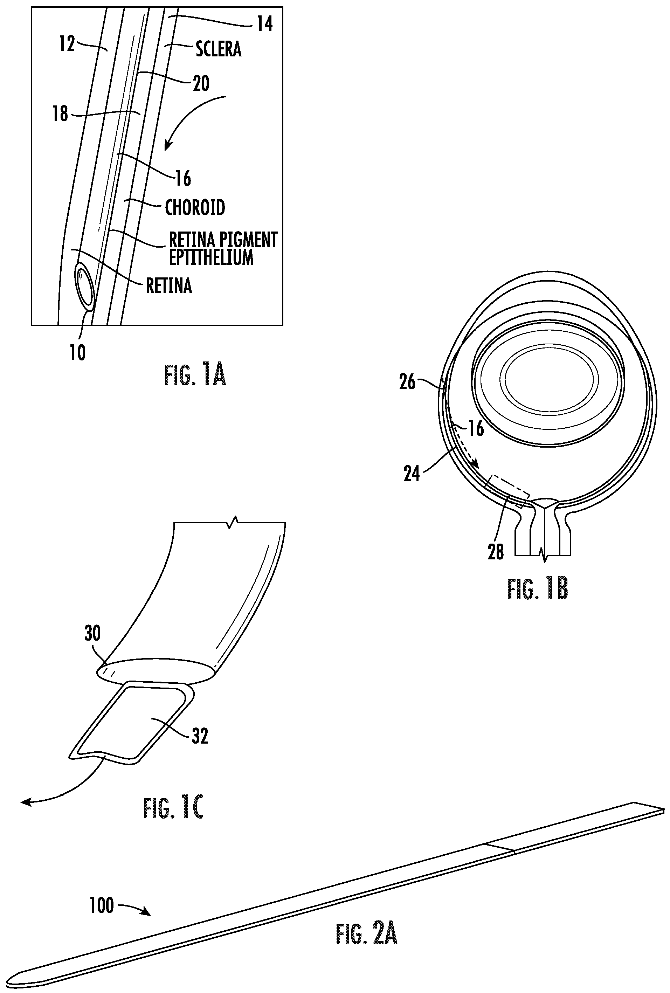

FIG. 1A illustrates a partially sectional view of a plane of delivery, according to an embodiment of the present invention. As illustrated in FIG. 1A, a delivery path 10 extends between the retina 12 and the sclera 14 in the subretinal space 16. The sclera 14, choroid 18, and the retina pigment epithelium 20 are to one side of the device and the retina 12 is to the other side of the device. The subretinal space 16 can be accessed through the scleral side or the subretinal side. Visualization of the entry point can be done with an optical coherence tomography (OCT) sensor, which allows for the user to determine the depth of penetration of the device at entry. The OCT sensor is mounted or placed at or near the tip of the device Visualization can continue along the delivery path to the target point, in some embodiments of the present invention, if desired.

FIG. 1B illustrates a partially sectional view of an entry point and a target point, according to an embodiment of the present invention. The device of the present invention should travel along a curved trajectory 24 in the subretinal space 16, between the entry point 26 and the target point 28. As noted with respect to FIG. 1A, the entry point 26 can be accessed through the scleral side or the retinal side. The curved trajectory 24 in the subretinal space 16 avoids trauma to the retina, retinal pigment epithelium, and choroid.

FIG. 1C illustrates a side view of an injection cartridge tip, according to an embodiment of the present invention. The injection cartridge tip 30 is advanced to the target site, as described with respect to FIGS. 1A and 1B. The injection cartridge tip 30 thereby delivers material or cells 32 to the target area, in a manner that is minimally traumatic to the target area and the material or cells 32 to be delivered. The device and the injection cartridge tip 30 of the device are configured to inject a planar sheet or other configuration of material or cells 32. For delivery of stem cells, this allows for the delivery of the stem cells in the correct apical-basal orientation. One design to achieve this is by an elliptical cross section of the injection cartridge tip 30.

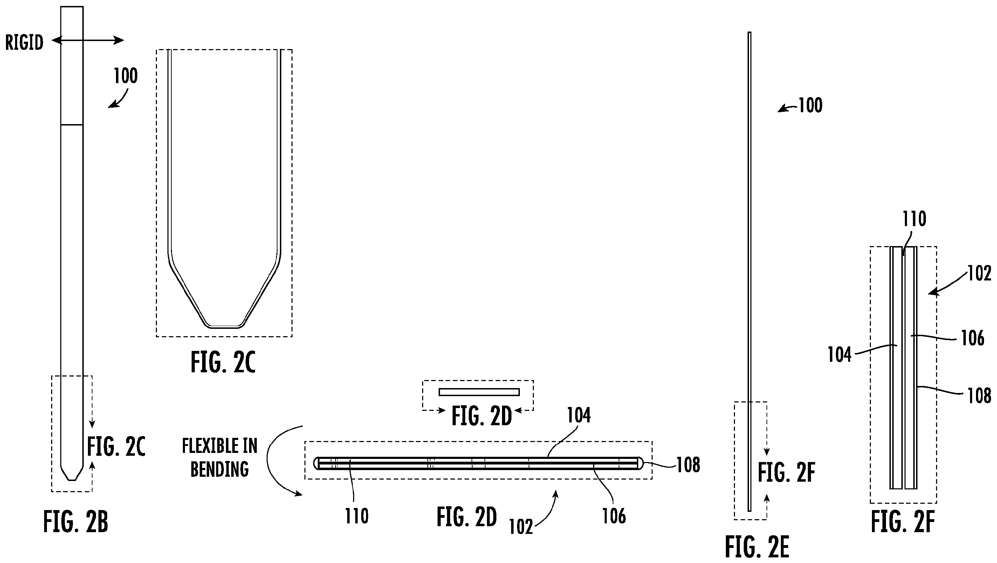

FIGS. 2A-2F illustrate views of a device for delivery of material or cells to the retina, according to an embodiment of the present invention. FIG. 2A illustrates a perspective view of a device for delivery of cells to the retina, according to an embodiment of the present invention. FIG. 2B illustrates a top-down view of a device for delivery of cells to the retina, according to an embodiment of the present invention. FIG. 2C illustrates an enlarged view of a distal end of the device for delivery of cells to the retina of FIG. 2B, according to an embodiment of the present invention. FIG. 2D illustrates a front view of a distal end of the device for delivery of cells to the retina of FIG. 2B, according to an embodiment of the present invention. FIG. 2E illustrates a side view of a device for delivery of cells to the retina, according to an embodiment of the present invention. FIG. 2F illustrates an enlarged view of a distal end of the device for delivery of cells to the retina of FIG. 2E, according to an embodiment of the present invention. With respect to FIGS. 2A-2F, the device 100 includes a tube 102. The tube includes two flat strips 104, 106 jacketed by a thin layer of elastic material 108. The two flat strips 104, 106 are formed from a flexible material that allows the device 100 to curve along the subretinal space. In a preferred embodiment of the present invention, the two flat strips 104, 106 are formed from polyimide and the surrounding thin layer of elastic material 108 is formed from latex. A thin, flat lumen 110 is defined between the two flat strips 104, 106 and allows for dispensing material or cells through the device. While a thin flat lumen is described as an example herein, this is merely one embodiment, and any lumen shape known to or conceivable by one of skill in the art could also be used. The geometry of the device 100 allows it be extremely flexible in one bending direction, but more rigid in the other direction. The multilayer structure and elastic outer jacket allows objects to be passed through the tube.

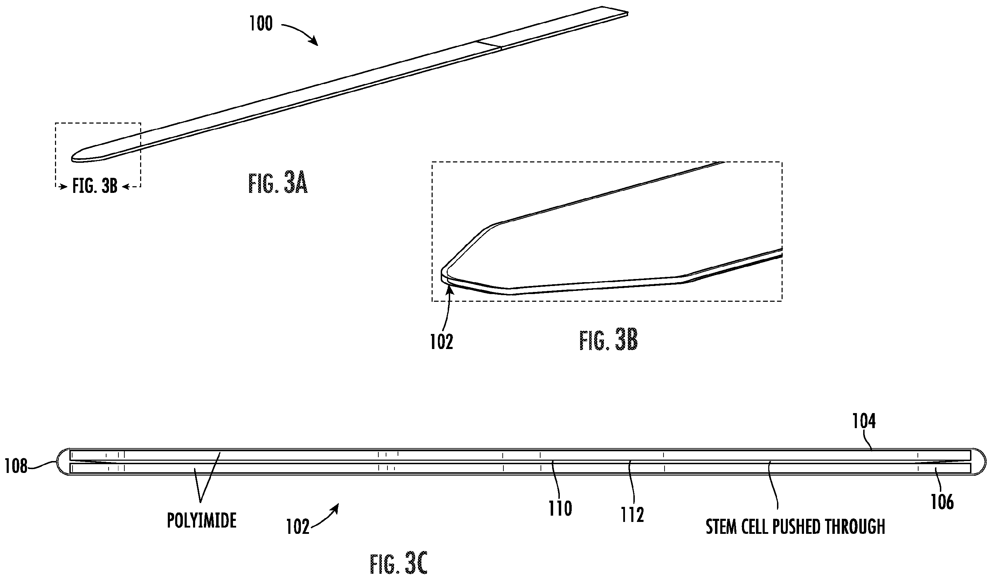

FIGS. 3A-3C illustrate views of a device for delivery of material or cells to the retina. FIG. 3A illustrates a perspective view of a device for delivery of cells to the retina, according to an embodiment of the present invention. FIG. 3B illustrates an enlarged view of a distal end of the device for delivery of cells to the retina of FIG. 3A, according to an embodiment of the present invention. FIG. 3C illustrates an enlarged view of a distal end of the device for delivery of cells to the retina of FIG. 3B, according to an embodiment of the present invention. As illustrated in FIGS. 3A-3C, the device 100 includes a tube 102. The tube 102 includes two flat strips 104, 106 jacketed by a thin layer of elastic material 108. The two flat strips 104, 106 are formed from a flexible material that allows the device 100 to curve along the subretinal space. In a preferred embodiment of the present invention, the two flat strips 104, 106 are formed from polyimide and the surrounding thin layer of elastic material 108 is formed from latex. A thin, flat lumen 110 is defined between the two flat strips 104, 106 and allows for dispensing material or cells through the device. As illustrated in FIG. 3C material or cells 112 can be pushed through the space defined by the two flat strips 104, 106. The tube 102, as configured, is intended to be slid into the sub-retinal space to allow delivery of treatment for various diseases or disorders. The current protocol is to make an incision in the sclera and choroid to access the subretinal space. The tube 102 is then inserted into the space between the choroid and retina. Because of its flexibility, the tube 102 can be manually inserted and will conform to the curvature of the eye. After the tube is in place, the thin layer of elastic material 108, preferably latex, protects the delicate tissues of the retina and choroid, while the polyimide forming the two flat strips 104, 106 allows the easy passage of objects between the two polyimide layers. The leading edge of the device is rounded to help separate the retina and choroid with minimal trauma.

The device described above can be used in conjunction with a system for transfer or material or cells to the retina. The system can include an optical coherence tomography (OCT) imaging device. The system can include lubricating material between the components to facilitate smooth passage and prevent unwanted adhesion of the material to be delivered. The system can also include a hub for facilitating the injection of material or cells to the retina. The hub can include a reservoir for the material or cells, such as a syringe or other reservoir known to or conceivable to one of skill in the art.

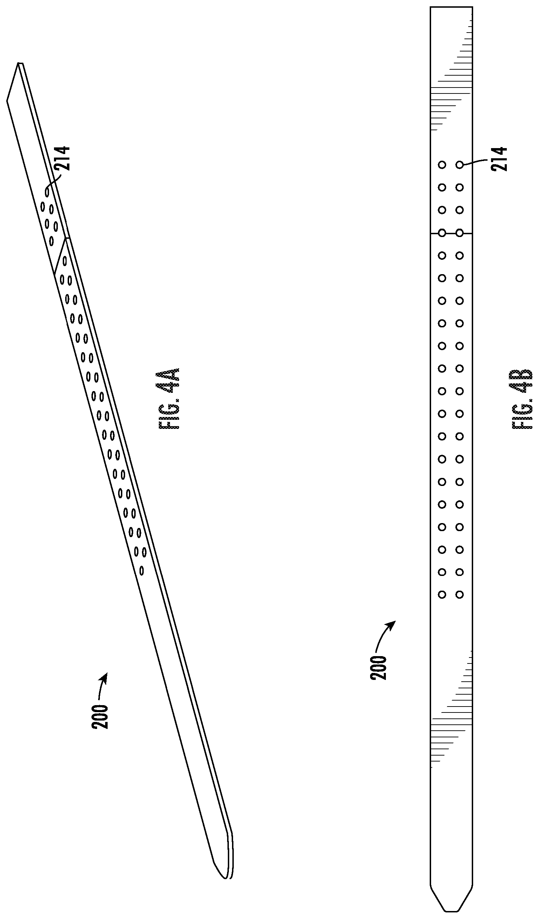

FIGS. 4A and 4B illustrate views a device for delivery of material or cells to the retina, according to an embodiment of the present invention. FIG. 4A illustrates a perspective view of the device 200 and FIG. 4B illustrates a top down view of the device 200. As illustrated in FIGS. 4A and 4B, the device 200 includes fixation holes 214 along each edge of the strips of flexible material that are used to secure the device to the eye during the insertion procedure.

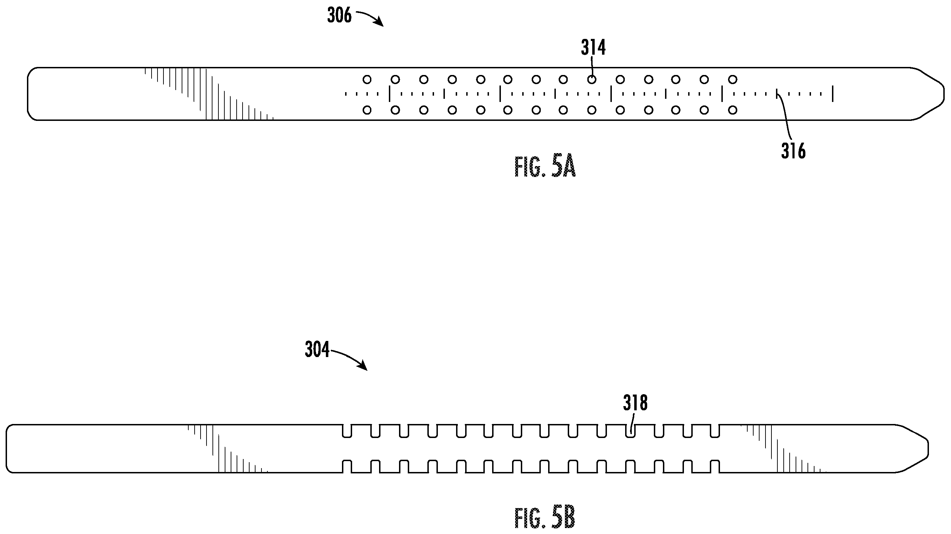

FIG. 5A illustrates a top down view of a bottom layer of a device for delivery of material or cells to the retina, according to an embodiment of the present invention. FIG. 5B illustrates a top down view of a top layer of a device for delivery of material or cells to the retina, according to an embodiment of the present invention. FIG. 5A illustrates the bottom layer 306 of the device. The bottom layer 306 includes fixation holes 314 along a length of the strip. The bottom later also includes marks 316 at 1 mm intervals to allow the insertion length to be easily measured. The marks 316 begin approximately 10 mm from the tip of the device. FIG. 5B illustrates the top layer 304 of the device. The top layer includes cutouts 318. The addition of cutouts 318 on the top layer 304 to aid in the separation of layers after the bottom layer 306 is fixed to the surface with sutures. The two flat strips 304, 306 are formed from a flexible material that allows the device to curve along the subretinal space. In a preferred embodiment of the present invention, the two flat strips 304, 306 are formed from polyimide. Alternately, the two flat strips can be formed from any suitable material known to or conceivable by one of skill in the art. The top and bottom layers 304, 306 are approximately 0.025 mm (0.001 inches) thick.

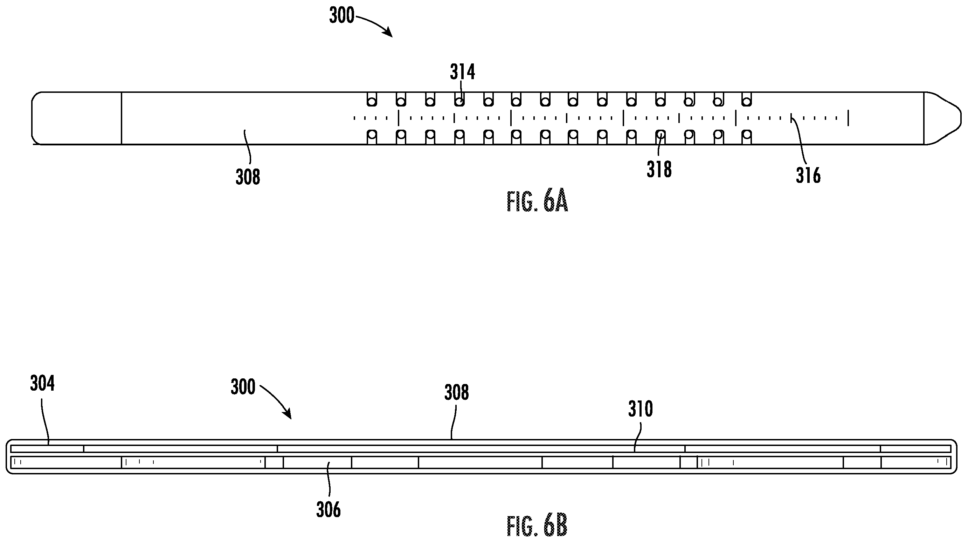

FIG. 6A illustrates a top down view of a device for delivery of material or cells to the retina, according to an embodiment of the present invention. The device 300, illustrated in FIG. 6A, includes a jacket of a thin layer of elastic material 308. The elastic material can take the form of latex. Any other suitable material or latex substitute known to or conceivable to one of skill in the art can also be used. FIG. 6A also shows the fixation holes 314 and cutouts 318. Marks 316 are also included to allow the distance the device 300 is inserted to be measured. FIG. 6B illustrates an end view of the device for delivery illustrated in FIG. 6A. FIG. 6B shows two flat strips 304, 306 jacketed by a thin layer of elastic material 308. The two flat strips 304, 306 are formed from a flexible material that allows the device 300 to curve along the subretinal space. In a preferred embodiment of the present invention, the two flat strips 304, 306 are formed from polyimide and the surrounding thin layer of elastic material 308 is formed from latex. A thin, flat lumen 310 is defined between the two flat strips 304, 306 and allows for dispensing material or cells through the device. In FIGS. 6A and 6B the top and bottom strips are encased in latex tube. The latex tube starts at the end of the shoulder of the tip and extends to approximately 10mm from the proximal end. Flexible cyanoacrylate adhesive (Loctite 4902) is used to adhere the distal ends of the polyimide layers to the latex. Primer is used to improve bond quality (Loctite 7701). Any other suitable way to construct the device 300 known to or conceivable by one of skill in the art can also be used.

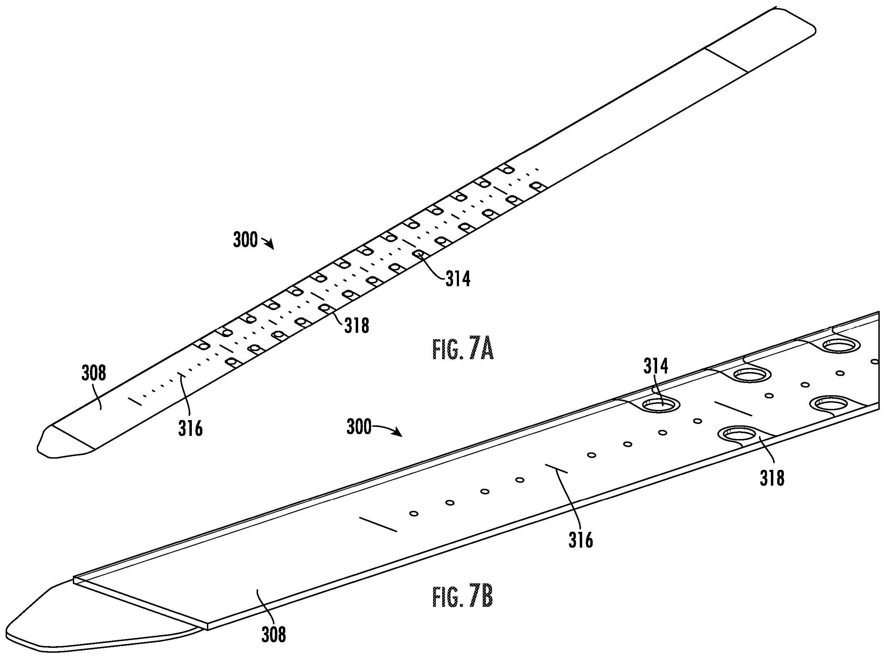

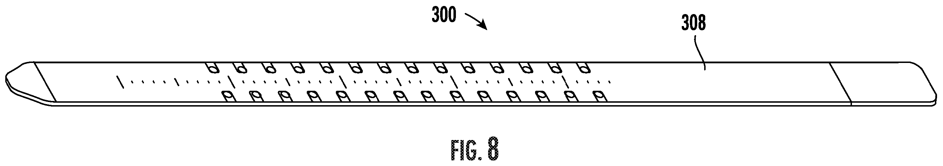

FIGS. 7A and 7B illustrate perspective views of a device for delivery of material or cells to the retina, according to an embodiment of the present invention. FIG. 8 illustrates a perspective view of a device for delivery of material or cells to the retina, according to an embodiment of the present invention. FIGS. 7A and 7B and FIG. 8 show the device 300 as well as the fixation holes 314, cutouts 318, and marks 316 in greater detail. The thin layer of elastic material 308 is also illustrated. In a preferred embodiment, the leading edge of the device is at least slightly rounded to help separate the retina and choroid with minimal trauma.

In an exemplary embodiment, to form the thin layer of elastic material in a preferred embodiment, Mehron liquid latex is mixed with distilled water in a 3:1 ratio (by weight). The latex mixture is (partially) degassed prior to dip coating to eliminate bubbles in the finished tubes. Latex tubes are created by dip coating 3mm diameter glass tubes. A single layer created at room temperature. The tubes are cured at 50°C for 2 hours and then set at room temperature for 12 hours. The tubes are carefully removed from the tubes just prior to use by using a mixture of water and mild soap. Wall thickness is approximately 0.02-0.05mm. This method of forming the thin layer of elastic material is included by way of example and is not meant to be considered limiting. Any other suitable method of forming the thin, elastic layer known to or conceivable to one of skill in the art can also be used.

The present invention is carried out using a computer, non-transitory computer readable medium, or alternately a computing device or non-transitory computer readable medium incorporated into the scanner. Indeed, any suitable method of calculation known to or conceivable by one of skill in the art could be used. It should also be noted that while specific equations are detailed herein, variations on these equations can also be derived, and this application includes any such equation known to or conceivable by one of skill in the art.

A non-transitory computer readable medium is understood to mean any article of manufacture that can be read by a computer. Such non-transitory computer readable media includes, but is not limited to, magnetic media, such as a floppy disk, flexible disk, hard disk, reel-to-reel tape, cartridge tape, cassette tape or cards, optical media such as CD-ROM, writable compact disc, magneto-optical media in disc, tape or card form, and paper media, such as punched cards and paper tape. The computing device can be a special computer designed specifically for this purpose. The computing device can be unique to the present invention and designed specifically to carry out the method of the present invention. Imagers, such as the optical coherence tomography sensor described herein, generally have a console, which is a proprietary master control center of the scanner designed specifically to carry out the operations of the imaging and receive the imaging data created by the sensor. Typically, this console is made up of a specialized computer, custom keyboard, and multiple monitors. There can be two different types of control consoles, one used by the OCT operator and the other used by the physician. The operator's console controls such variables as the thickness of the image, the distances to retinal layers, and needle tip position. The physician's viewing console allows viewing of the images without interfering with the normal OCT imaging operation. This console is capable of image analysis. The operating console computer is a non-generic computer specifically designed for bilateral (input output) communication with the imager. It is not a standard business or personal computer that can be purchased at a local store. Additionally this console computer carries out communications with the imager through the execution of proprietary custom built software that is designed and written for the computer hardware to specifically operate the imager hardware.

The many features and advantages of the invention are apparent from the detailed specification, and thus, it is intended by the appended claims to cover all such features and advantages of the invention which fall within the scope of the invention as defined in the claims..

## Claims

1. A device for delivery of material or cells to a retinal space comprising:
a stacked pair of thin, elongate strips of flexible material (104,106);
a thin layer of elastic material (108) surrounding the stacked pair of thin, elongate strips of flexible material;
wherein the stacked pair of thin, elongate strips of flexible material and thin layer of elastic material surrounding the stacked pair of thin, elongate strips of flexible material form a flexible, expandable tube (102), wherein an elongate lumen (110) extends between the stacked pair of thin, elongate strips of flexible material and
wherein the elongate lumen is configured for dispensing the material or cells through the device..

2. The device of claim 1 wherein the stacked pair of thin, elongate strips of flexible material are formed from a flexible material that allows the device to curve along the subretinal space.

3. The device of claim 1 wherein a top strip of the stacked pair of thin, elongage strips includes cutouts (318) to aid in a separation of layers.

4. The device of claim 1 wherein a bottom strip of the stacked pair of thin, elongate strips include markings (316) to allow a length of insertion to be measured.

5. The device of claim 1 wherein a geometry of the device is configured such that the device is extremely flexible in one bending direction, but more rigid in the other direction.

6. The device of claim 1 further comprising an optical coherence tomography sensor for visualization of an entry point of the device, such that a user can determine depth of penetration of the device at entry.

7. The device of claim 1 further comprising a distal end of the stacked pair of thin, elongate strips of flexible material having a rounded shape, or wherein the stacked pair of thin elongate strips of flexible material further define holes (314) for fixation of the device to any eye.

8. The device of claim 1 wherein the device is configured to move through a subretinal space of an eye, or wherein the device is configured to deliver cells to a retina.

9. The device of claim 1 wherein the stacked pair of thin, elongate strips of flexible material are formed from a material that allows the passage of objects between the stacked pair of thin, elongate strips of flexible material.

10. The device of claim 1 further comprising a lubricating material disposed between the stacked pair of thin, elongate strips of flexible material to facilitate smooth passage and prevent adhesion of the material to be delivered.

11. The device of claim 1 further comprising a hub for facilitating the injection of material or cells to the retina,
optionally wherein the hub can include a reservoir for the material or cells,
and optionally wherein the reservoir takes the form of a syringe.

## Patentansprüche

1. Eine Vorrichtung zum Zuführen von Material oder Zellen in einen Netzhautraum, die Folgendes beinhaltet:
ein gestapeltes Paar dünner, länglicher Streifen aus flexiblem Material (104, 106);
eine dünne Schicht aus elastischem Material (108), die das gestapelte Paar dünner, länglicher Streifen aus flexiblem Material umgibt;
wobei das gestapelte Paar dünner, länglicher Streifen aus flexiblem Material und die dünne Schicht aus elastischem Material, die das gestapelte Paar dünner, länglicher Streifen aus flexiblem Material umgibt, einen flexiblen, ausdehnbaren Schlauch (102) bilden, wobei sich ein längliches Lumen (110) zwischen dem gestapelten Paar dünner, länglicher Streifen aus flexiblem Material erstreckt und wobei das längliche Lumen zum Abgeben des Materials oder der Zellen durch die Vorrichtung konfiguriert ist.

2. Vorrichtung gemäß Anspruch 1, wobei das gestapelte Paar dünner, länglicher Streifen aus flexiblem Material aus einem flexiblen Material gebildet ist, das es der Vorrichtung ermöglicht, sich entlang des subretinalen Raums zu krümmen.

3. Vorrichtung gemäß Anspruch 1, wobei ein oberer Streifen des gestapelten Paars dünner, länglicher Streifen Ausschnitte (318) umfasst, um eine Trennung von Schichten zu unterstützen.

4. Vorrichtung gemäß Anspruch 1, wobei ein unterer Streifen des gestapelten Paars dünner, länglicher Streifen Markierungen (316) umfasst, um zu ermöglichen, dass eine Einführungslänge gemessen wird.

5. Vorrichtung gemäß Anspruch 1, wobei eine Geometrie der Vorrichtung so konfiguriert ist, dass die Vorrichtung in einer Biegerichtung extrem flexibel, aber in der anderen Richtung steifer ist.

6. Vorrichtung gemäß Anspruch 1, die ferner einen Sensor für optische Kohärenztomographie zur Visualisierung eines Eintrittspunkts der Vorrichtung beinhaltet, so dass ein Benutzer die Eindringtiefe der Vorrichtung beim Eintritt bestimmen kann.

7. Vorrichtung gemäß Anspruch 1, die ferner ein distales Ende des gestapelten Paars dünner, länglicher Streifen aus flexiblem Material mit einer abgerundeten Form beinhaltet, oder wobei das gestapelte Paar dünner, länglicher Streifen aus flexiblem Material ferner Löcher (314) zur Befestigung der Vorrichtung an einem beliebigen Auge definiert.

8. Vorrichtung gemäß Anspruch 1, wobei die Vorrichtung konfiguriert ist, um sich durch einen subretinalen Raum eines Auges zu bewegen, oder wobei die Vorrichtung konfiguriert ist, um Zellen in eine Netzhaut zuzuführen.

9. Vorrichtung gemäß Anspruch 1, wobei das gestapelte Paar dünner, länglicher Streifen aus flexiblem Material aus einem Material gebildet ist, das den Durchgang von Objekten zwischen dem gestapelten Paar dünner, länglicher Streifen aus flexiblem Material ermöglicht.

10. Vorrichtung gemäß Anspruch 1, die ferner ein Schmiermaterial beinhaltet, das zwischen dem gestapelten Paar dünner, länglicher Streifen aus flexiblem Material angeordnet ist, um einen glatten Durchgang zu erleichtern und das Anhaften des zuzuführenden Materials zu verhindern.

11. Vorrichtung gemäß Anspruch 1, die ferner einen Ansatz zum Erleichtern der Injektion von Material oder Zellen in die Netzhaut beinhaltet,
wobei der Ansatz optional ein Reservoir für das Material oder die Zellen umfassen kann und
wobei das Reservoir optional die Form einer Spritze annimmt.

## Revendications

1. Un dispositif pour la mise en place de matériau ou de cellules dans un espace rétinien comprenant :
une paire superposée de minces bandes allongées de matériau flexible (104, 106) ;
une mince couche de matériau élastique (108) entourant la paire superposée de minces bandes allongées de matériau flexible ;
dans lequel la paire superposée de minces bandes allongées de matériau flexible et la mince couche de matériau élastique entourant la paire superposée de minces bandes allongées de matériau flexible forment un tube flexible, expansible (102), dans lequel une lumière allongée (110) s'étend entre la paire superposée de minces bandes allongées de matériau flexible et dans lequel la lumière allongée est configurée pour dispenser le matériau ou les cellules à travers le dispositif.

2. Le dispositif de la revendication 1 dans lequel la paire superposée de minces bandes allongées de matériau flexible sont formées à partir d'un matériau flexible qui permet au dispositif de se courber le long de l'espace sous-rétinien.

3. Le dispositif de la revendication 1 dans lequel une bande de dessus de la paire superposée de minces bandes allongées inclut des découpes (318) pour aider à une séparation de couches.

4. Le dispositif de la revendication 1 dans lequel une bande de dessous de la paire superposée de minces bandes allongées inclut des marquages (316) pour permettre de mesurer une longueur d'insertion.

5. Le dispositif de la revendication 1 dans lequel une géométrie du dispositif est configurée de telle sorte que le dispositif est extrêmement flexible dans une direction de flexion, mais plus rigide dans l'autre direction.

6. Le dispositif de la revendication 1 comprenant en sus un capteur de tomographie en cohérence optique pour la visualisation d'un point d'entrée du dispositif, de telle sorte qu'un utilisateur peut déterminer la profondeur de pénétration du dispositif au niveau de l'entrée.

7. Le dispositif de la revendication 1 comprenant en sus le fait qu'une extrémité distale de la paire superposée de minces bandes allongées de matériau flexible a une forme arrondie, ou dans lequel la paire superposée de minces bandes allongées de matériau flexible définissent en sus des trous (314) pour la fixation du dispositif à n'importe quel œil.

8. Le dispositif de la revendication 1 dans lequel le dispositif est configuré pour se déplacer à travers un espace sous-rétinien d'un œil, ou dans lequel le dispositif est configuré pour mettre en place des cellules dans une rétine.

9. Le dispositif de la revendication 1 dans lequel la paire superposée de minces bandes allongées de matériau flexible sont formées à partir d'un matériau qui permet le passage d'objets entre la paire superposée de minces bandes allongées de matériau flexible.

10. Le dispositif de la revendication 1 comprenant en sus un matériau lubrifiant disposé entre la paire superposée de minces bandes allongées de matériau flexible afin de faciliter le passage en douceur et empêcher l'adhérence du matériau à mettre en place.

11. Le dispositif de la revendication 1 comprenant en sus un moyeu destiné à faciliter l'injection de matériau ou de cellules dans la rétine,
facultativement dans lequel le moyeu peut inclure un réservoir pour le matériau ou les cellules, et
facultativement dans lequel le réservoir se présente sous la forme d'une seringue.
